# EUROPÄISCHE PATENTANMELDUNG

(11) **EP 2 395 355 A1**
(43) Veröffentlichungstag der Anmeldung: **14.12.2011**
(21) Anmeldenummer: 11169038.4
(22) Anmeldetag: 08.06.2011
(51) Int. Cl.: G01N 33/53, G01N 33/68, G01N 33/542, G01N 33/573

(54) **Homogener Aktivitätstest zur Bestimmung von enzymatischen Reaktionen**

(30) Priorität: 10.06.2010 EP 10005971
(71) Anmelder: Siemens Healthcare Diagnostics Products GmbH, 35041 Marburg (DE)
(72) Erfinder: Schwarz, Herbert, 35102 Lohra (DE); Vitzthum, Frank, Dr., 35094 Lahntal (DE); Dekevic, Katharina, 70437 Stuttgart-Rot (DE)

(57) **Zusammenfassung**

Die vorliegende Erfindung liegt auf dem Gebiet der in vitro Diagnostik und betrifft ein homogenes Verfahren zur Bestimmung der enzymatischen Aktivität eines Analyten in einer Probe. Das Verfahren ermöglicht ferner die simultane Bestimmung der Enzymmenge im gleichen Reaktionsansatz sowie die Bestimmung von Enzymaktivatoren oder -inhibitoren.

## Beschreibung

Die vorliegende Erfindung liegt auf dem Gebiet der in vitro Diagnostik und betrifft ein homogenes Verfahren zur Bestimmung von enzymatisch katalysierten Reaktionen in einer Probe. Mit der erfindungsgemäßen Bestimmung von enzymatisch katalysierten, chemischen Reaktionen ist es möglich, den Aktivitätszustand von Enzymen oder das Vorhandensein von Substanzen, die den Aktivitätszustand beeinflussen, zu bestimmen.

Eine chemische Reaktion ist ein Vorgang, bei dem Substanzen umgewandelt werden. Aus einem oder mehreren Ausgangsstoffen (Edukte oder Reaktanten) entstehen ein oder mehrere Reaktionsprodukte. Das Produkt hat andere chemische oder physikalische Eigenschaften als das Edukt.

Bei einer enzymatisch katalysierten Reaktion wird die Einleitung, Beschleunigung oder Lenkung der chemischen Reaktion durch Beteiligung eines Enzyms durchgeführt.

Bei vielen diagnostisch relevanten Parametern handelt es sich um Enzyme, Reaktanten, Reaktionsprodukte oder Enzymaktivitätsmodulatoren. Unter einem Aktivitätsmodulator ist eine Substanz zu verstehen, die als Inhibitor, Aktivator, Agonist, Antagonist oder Cofaktor die Aktivität eines Enzyms zu beeinflussen vermag.

Ein Beispiel für ein diagnostisch relevantes Enzym ist Thrombin, ein Blutgerinnungsfaktor aus der Gruppe der Serinproteasen, dessen Aktivitätsbestimmung in einer Plasmaprobe eines Patienten Aussagen über den Status der Blutgerinnung des Patienten ermöglicht. Ein anderes Beispiel ist die den Blutgerinnungsfaktor VII aktivierende Protease (FSAP), deren Aktivitätsbestimmung z.B. Aussagen ermöglicht über das Risiko eines Patienten, eine Thrombose zu entwickeln.

Die Aktivität eines Enzyms wird üblicherweise über die Umsetzung eines mehr oder weniger spezifischen Substrats bestimmt. Bei den bekannten Verfahren werden homogene Verfahren (ohne Trennschritte) und heterogene Verfahren (mit einem oder mehreren Trennschritten) unterschieden.

Bei heterogenen Verfahren wird die nachzuweisende Substanz angereichert. Das heißt, die zu untersuchende Probe wird einem Verfahren unterzogen, das dazu führt, dass andere Probenbestandteile in ihrer Konzentration relativ zu der nachzuweisenden Substanz reduziert oder vollständig entfernt werden. Beispielsweise werden hierfür Affinitätsverfahren eingesetzt.

Im Gegensatz hierzu werden bei homogenen Verfahren die Proben direkt im Testansatz untersucht. Trennverfahren wie beispielsweise Waschschritte kommen nicht zum Einsatz.

Beispiele für homogene Verfahren zur Bestimmung der Aktivität eines Enzyms sind z.B. Thrombinaktivitätsteste, wie der ETP-Test, wie in EP-A2-0420332 beschrieben. Das Thrombin einer Plasmaprobe wird aktiviert, und es wird ein chromogenes Peptidsubstrat mit der Probe vermischt, dessen spezifische Spaltung durch Thrombin photometrisch gemessen wird. Ein solches homogenes Testformat eignet sich jedoch nicht für die Bestimmung der Aktivität von Enzymen mit niedriger Substratspezifität, da durch die mangelnde Substratspezifität auch eine Umsetzung des Substrats durch andere Enzyme aus der Probe stattfinden kann. Auch für die Bestimmung von Enzymen, für die ausschließlich Substrate mit mangelnder analytischer Sensitivität zur Verfügung stehen, sind homogene Verfahren nicht geeignet. Ferner ist ein solches homogenes Testformat in der Regel auch nicht geeignet für die Bestimmung der Aktivität von Enzymen mit niedriger Volumenaktivität.

Die Volumenaktivität beschreibt die katalytische Aktivität eines Enzyms pro Volumen. Die katalytische Aktivität ist durch die Menge an Substrat, die pro Zeiteinheit umgesetzt wird, oder die Menge an Produkt, die pro Zeiteinheit gebildet wird, definiert. Die Volumenaktivität wird häufig in µmol (Substratabbau oder Produktbildung) pro Minute und Liter angegeben, d.h. µmol/min*L. Für µmol/min wird üblicherweise der Begriff Unit (U) oder katalytische Unit (kU) verwendet. Nach dem International System of Units (SI) sollte die Einheit Katal (kat) verwendet werden, d.h. mol/sec.

Neben der Volumenaktivität eines Enzyms in einer Probe beschreibt insbesondere die spezifische Aktivität eines Enzyms dessen Aktivitätszustand. Die spezifische Aktivität ergibt sich aus dem Verhältnis der katalytischen Aktivität eines Enzyms pro Menge des vorhandenen Enzyms, d.h. µmol/min*g (U/g). Die spezifische Aktivität kann beispielsweise Auskunft über den Aktivierungsgrad, den Degradationsgrad oder die Inhibition oder Aktivierung eines Enzyms liefern.

Wenn in einer zu untersuchenden Probe andere Enzyme vorhanden sind, die das eingesetzte Substrat ebenfalls umsetzen, was insbesondere in komplexen Proben wie Körperflüssigkeitsproben der Fall sein kann, ist eine spezifische Bestimmung der gewünschten Enzymaktivität ohne ein geeignetes hochspezifisches Substrat direkt in der Probe nicht möglich.

Im Stand der Technik kann dieses Problem dadurch gelöst werden, dass beispielsweise statt der Enzymaktivität die Enzymkonzentration bzw. Antigenkonzentration mittels eines spezifischen Tests, beispielsweise mittels eines Immunoassays bestimmt wird. Die Antigenkonzentration wird mit der zu erwartenden Enyzmaktivität korreliert. Die Abschätzung der Enzymaktivität durch die Enzymkonzentration ist ungeeignet, wenn der Aktivitätszustand des Enzyms beispielsweise durch Aktivitätsmodulatoren beeinflusst wird. Damit kann die Aktivität eines Enzyms oder dessen Aktivitätszustand nicht immer sicher ermittelt werden.

Des Weiteren wird im Stand der Technik das Problem üblicherweise auch durch den Einsatz von heterogenen Testsystemen gelöst. Hierbei wird das nachzuweisende Enzym von den übrigen Probenbestandteilen abgetrennt, bevor die Enzymaktivität bestimmt wird. Die Abtrennung erfolgt üblicherweise durch das Inkontaktbringen der Probe mit einer Festphase, die eine spezifische Bindungsaffinität zu dem nachzuweisenden Enzym aufweist, und der anschließenden Separation der Festphase.

Der Nachteil solcher heterogener Verfahren besteht darin, dass sie mindestens einen Separationsschritt, wenn nicht sogar weitere Waschschritte, umfassen. Diese zusätzlichen Verfahrensschritte erschweren die Automatisierung und verlängern die Bearbeitungszeit. Die Bearbeitungszeit verlängert sich auch dadurch, dass die Probe für einen gewissen Zeitraum mit der Festphase inkubiert werden muss, um die Bindung des nachzuweisenden Enzyms an die Festphase zu ermöglichen. Ein weiterer Nachteil besteht darin, dass durch die Inkubation, Separation und das Waschen der Festphase die Aktivität des nachzuweisenden Enzyms beeinflusst werden kann. Beispielsweise können Konformationsänderungen oder Degradierung des Enzyms zu Aktivitätsänderungen führen. Ein weiterer Nachteil der heterogenen Verfahren besteht darin, dass andere Probenbestandteile abgetrennt oder zumindest deren Konzentrationen reduziert werden, so dass probenintrinsische Bindungspartner, Cofaktoren, Aktivatoren oder Inhibitoren, welche die Aktivität des nachzuweisenden Enzyms beeinflussen, ebenfalls abgetrennt werden, so dass keine Bestimmung der Enzymaktivität oder etwaiger Bindungspartner des Enzyms im physiologischen Kontext der Probenmatrix möglich ist.

Der vorliegenden Erfindung lag daher die Aufgabe zugrunde, ein homogenes Verfahren bereit zu stellen, das die spezifische Bestimmung der Aktivität eines Enzyms auch in Gegenwart von störenden Enzymen ermöglicht, die das verwendete Substrat ebenfalls umsetzen. Des Weiteren lag die Aufgabe zugrunde den Aktivitätszustand eines Enzyms zu bestimmen, d.h. abgesehen von der Volumenaktivität auch einen Hinweis auf die Enzymkonzentration und damit die spezifische Aktivität oder das Vorhandensein von Aktivitätsmodulatoren zu erhalten. Insbesondere sollte eine Bestimmung, vorzugsweise eine quantitative Bestimmung der spezifischen Aktivität oder von Aktivitätsmodulatoren ermöglicht werden. Ferner soll die Bestimmung der Aktivität eines Enzyms ermöglicht werden, das in niedriger Volumenaktivität vorliegt.

Diese Aufgabe wird dadurch gelöst, dass der zu untersuchenden Probe ein Substrat sowie eine erste und eine zweite Komponente eines signalbildenden Systems zugegeben wird, wobei die erste Komponente des signalbildenden Systems mit dem Substrat assoziiert ist oder wird und wobei die zweite Komponente des signalbildenden Systems mit dem zu bestimmenden Enzym assoziiert wird. Das signalbildende System ist so beschaffen, dass ein detektierbares Signal nur entsteht, wenn die erste und die zweite Komponente des signalbildenden Systems durch Bindung des zu bestimmenden Enzyms an das Substrat in räumliche Nähe zueinander gebracht werden. Die Bindung etwaiger anderer störender Enzyme an das Substrat hat nicht zur Folge, dass ein Signal generiert wird, weil ausschließlich das nachzuweisende Enzym mit der zweiten Komponente des signalbildenden Systems assoziiert ist. Das enstehende Signal liefert Informationen über die Aktivität des Enzyms in der Probe.

Die vorliegende Erfindung betrifft ein Verfahren zur Bestimmung der Aktivität eines Enzyms in einer Probe. Unter dem Begriff "Enzym" ist im Sinne der vorliegenden Erfindung jede Substanz zu verstehen, die eine Reaktion zu katalysieren vermag, ohne dabei verbraucht zu werden. Der Begriff "Enzym" umfasst Proteine sowie Nukleinsäuren wie z.B. katalytisch aktive RNA-Moleküle, sogenannte Ribozyme. Je nach Funktion wird ein Enzym im Wesentlichen einer der folgenden Klassen zugerechnet: Oxidoreduktasen, Transferasen, Hydrolasen, Lyasen, Isomerasen oder Ligasen. Im Besonderen umfasst der Begriff "Enzym" die Blutgerinnungsfaktoren Faktor II, Faktor VII, Faktor IX, Faktor X, Faktor XI, Faktor XII, Faktor XIII, Protein C, die Faktor VII aktivierende Protease (FSAP), Präkallikrein, Plasminogen, Tissue-Plasminogenaktivator (t-PA), Prourokinase.

Unter dem Begriff "Probe" ist im Sinne der vorliegenden Erfindung das Material zu verstehen, das das nachzuweisende Enzym vermutlich enthält. Der Begriff "Probe" umfasst biologische Flüssigkeiten oder Gewebe insbesondere von Menschen und Tieren wie Blut, Plasma, Serum sowie andere Körperflüssigkeiten, Ausscheidungen oder Extrakte. Gegebenenfalls muss eine Probe vorbehandelt werden, um das Enzym für das Nachweisverfahren zugänglich zu machen oder um störende Probenbestandteile zu entfernen. Solch eine Vorbehandlung einer Probe mag die Abtrennung und/oder Lyse von Zellen beinhalten, das Präzipitieren, die Hydrolyse oder die Denaturierung von Probenbestandteilen wie z.B. Proteinen, die Zentrifugation von Proben, das Behandeln der Probe mit organischen Lösungsmitteln wie z.B. Alkoholen, insbesondere Methanol; das Behandeln der Probe mit Detergenzien. Häufig wird die Probe in ein anderes, meistens wässriges, Medium überführt, welches mit dem Nachweisverfahren möglichst nicht interferieren soll.

Das erfindungsgemäße Verfahren umfasst die Bereitstellung eines Reaktionsgemisches enthaltend die Probe, mindestens ein Substrat, welches von dem zu bestimmenden Enzym gebunden und in mindestens ein Produkt umgesetzt werden kann und eine erste und eine zweite Komponente eines signalbildenden Systems, welche so zusammenwirken, dass ein detektierbares Signal entsteht, wenn die erste und die zweite Komponente des signalbildenden Systems in räumliche Nähe zueinander gebracht werden. Das Substrat ist oder wird mit der ersten Komponente des signalbildenden Systems assoziiert; das zu bestimmende Enzym wird mit der zweiten Komponente des signalbildenden Systems assoziiert. Infolge der Komplexbildung zwischen Enzym und Substrat werden die Komponenten des signalbildenden Systems in räumliche Nähe zueinander gebracht, wodurch ein detektierbares Signal entsteht, welches mit der Aktivität des Enzyms in der Probe korreliert.

Unter dem Begriff "Substrat" ist im Sinne der vorliegenden Erfindung eine Substanz zu verstehen, die vom Enzym gebunden und in mindestens ein Reaktionsprodukt umgesetzt wird. Bevorzugte Substrate, z.B. zum Nachweis von Peptidasen, die Peptidbindungen hydrolytisch spalten, bestehen aus Proteinen, Peptiden oder beinhalten zumindest einen Peptidanteil.

Unter dem Begriff "signalbildendes System" ist im Sinne der vorliegenden Erfindung ein System zu verstehen, das mindestens eine erste und eine zweite Komponente umfasst, welche so zusammenwirken, dass ein detektierbares Signal entsteht, wenn sie in räumliche Nähe zueinander gebracht werden und dadurch miteinander in Wechselwirkung treten können. Unter einer Wechselwirkung zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z.B. kurzlebigen SingulettSauerstoff - zu verstehen. Der Energietransfer kann von einer auf eine andere Komponente erfolgen, möglich ist aber auch eine Kaskade verschiedener Substanzen, über die der Energietransfer läuft. Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI^{®} Technologie) oder Photosensitizer und Fluorophor (WO 95/06877) oder radioaktives Iod<125> und Fluorophor (Udenfriend et al. (1985) Proc. Natl. Acad. Sci. 82:8672 - 8676) oder Fluorophor und Fluoreszenz-Quencher (US 3,996,345).

Die erste Komponente und/oder die zweite Komponente des signalbildenden Systems können mit einer partikulären Festphase kovalent oder über spezifische Wechselwirkung assoziiert oder in diese eingelagert sein. Unter dem Begriff partikuläre Festphase sind suspendierbare Teilchen zu verstehen, wie z.B. Metallsolen, Silica-Partikel, Magnetpartikel oder besonders bevorzugt Latexpartikel. Bevorzugt werden Partikel mit einem Durchmesser von 0,01 - 10 Mikrometer, insbesondere bevorzugt werden Partikel mit einem Durchmesser von 0,1 - 1 Mikrometer.

In einer Ausführungsform des erfindungsgemäßen Verfahrens weist das Substrat einen ersten Bindungspartner A eines ersten Bindungspaares A/B auf, und die erste Komponente des signalbildenden Systems weist den zweiten Bindungspartner B des Bindungspaares A/B auf, damit das Substrat mit der ersten Komponente des signalbildenden Systems assoziiert werden kann. Die Bindung des Substrats an die erste Komponente des signalbildenden Systems kann entweder während der Inkubation des Reaktionsgemisches, d.h. in Gegenwart von Probe und zweiter Komponente des signalbildenden Systems erfolgen, oder Substrat und erste signalbildende Komponente werden vor ihrer Zugabe zum Reaktionsgemisch separat inkubiert und der Komplex aus Substrat und erster Komponente des signalbildenden Systems wird zum Reaktionsgemisch gegeben.

Die Bindungspartner A und B sind zwei unterschiedliche Moleküle, die einander spezifisch erkennen und binden. Beispiele für spezifische Erkennung und Bindung sind Antikörper-Antigen-Wechselwirkungen, Enzym-Substrat-Wechselwirkungen, Polynukleotid-Wechselwirkungen etc.

Geeignete Bindungspaare A/B sind vor allem
Antigen/Antikörper-Kombinationen, wobei der Bindungspartner A ein antigenes Epitop des Substrats ist. Das antigene Epitop kann ein natürliches Sequenz- oder Strukturepitop eines natürlichen Proteins oder Proteinfragmentes sein. Das antigene Epitop kann auch ein heterologes Sequenz- oder Strukturepitop eines modifizierten Substrates sein. Beispiele für heterologe Sequenz- oder Strukturepitope sind FLAG-, oder HIS- oder Fluorescein-Tags, die insbesondere zur Markierung von Peptiden oder Proteinen verwendet werden. Weitere geeignete Bindungspaare A/B sind komplementäre Polynukleotide A und B. Der mit der ersten Komponente des signalbildenden Systems assoziierte Bindungspartner B ist so zu wählen, dass das Substrat spezifisch gebunden werden kann. Bevorzugterweise besteht der Bindungspartner B aus einem Antikörper oder einem Antigen-bindenden Fragment desselben. Besonders bevorzugte Bindungspaare A/B sind FLAG-Tag/anti-FLAG-Tag-Antikörper, HIS-Tag/anti-HIS-Tag-Antikörper Fluorescein/anti-Fluorescein-Antikörper, Biotin/Avidin und
Biotin/Streptavidin.

In einer anderen Ausführungsform des erfindungsgemäßen Verfahrens weist das zu bestimmende Enzym einen ersten Bindungspartner X eines zweiten Bindungspaares X/Y auf, und die zweite Komponente des signalbildenden Systems ist mit dem zweiten Bindungspartner Y des Bindungspaares X/Y assoziiert, damit das zu bestimmende Enzym durch Bindung der Bindungspartner X und Y während der Inkubation des Reaktionsgemisches an die zweite Komponente des signalbildenden Systems gebunden wird.

Die Bindungspartner X und Y sind zwei unterschiedliche Moleküle, die einander spezifisch erkennen und binden. X ist ein Enzym-spezifisches Struktur- oder Sequenzepitop, und Y ist -je nach Beschaffenheit des nachzuweisenden Enzyms- ein Antikörper oder Antikörperfragment oder ein Polynukleotid mit Spezifität für das Enzym-spezifische Struktur- oder Sequenzepitop.

Eine weitere Ausführungsform betrifft ein Verfahren zur Bestimmung der Aktivität eines proteolytischen Gerinnungsfaktors aus der Gruppe Faktor II, Faktor VII, Faktor IX, Faktor X, Faktor XI, Faktor XII und Protein C, wobei dem Reaktionsgemisch zusätzlich ein Agens zur direkten oder indirekten Aktivierung des proteolytischen Gerinnungsfaktors in der Probe zugegeben wird.

Zur direkten oder indirekten Aktivierung des proteolytischen Gerinnungsfaktors in der Probe wird die Probe üblicherweise mit einem Agens vermischt, das eine direkte oder indirekte Aktivierung des proteolytischen Gerinnungsfaktors bewirkt. Unter direkter Aktivierung ist zu verstehen, dass ein Agens verwendet wird, das den zu bestimmenden proteolytischen Gerinnungsfaktor direkt aktiviert, unabhängig von der Anwesenheit anderer Gerinnungsfaktoren. Unter indirekter Aktivierung ist zu verstehen, dass ein Agens verwendet wird, das einen oder mehrere Blutgerinnungsfaktoren der Blutgerinnungskaskade aktiviert, welche wiederum den zu untersuchenden proteolytischen Gerinnungsfaktor aktivieren. Die Art des Agens hängt davon ab, welcher Gerinnungsfaktor bestimmt werden soll, ob die Aktivität des Gerinnungsfaktors allein bestimmt werden soll oder ob die Funktionalität der Blutgerinnungskaskade oder eines Teilbereichs der Blutgerinnungskaskade (extrinsischer oder intrinsischer Weg) anhand eines Gerinnungsfaktors bestimmt werden soll. Substanzen und spezifische Mischungen verschiedener Substanzen, die eine direkte oder indirekte Aktivierung von proteolytischen Gerinnungsfaktoren ermöglichen, sind dem Fachmann hinlänglich bekannt und umfassen beispielsweise Phospholipide, wie z.B. negativ geladene Phospholipide; Lipoproteine, wie z.B. Thromboplastin; Proteine, wie z.B. Gewebefaktor, aktivierte Serinproteasen, wie z.B. Faktor IIa (Thrombin), Faktor VIIa, Faktor IXa, Faktor Xa, Faktor XIa, Faktor XIIa oder aktiviertes Protein C; Schlangengifte, wie z.B. PROTAC® Enzym, Ecarin, Textarin, Noscarin, Batroxobin, Thrombocytin oder Russell's Viper Venom (RVV); Kontaktaktivatoren, wie z.B. Silica, Kaolin, Ellagsäure oder Celite. Weitere Substanzen, die ein Agens enthalten kann, sind z.B. Puffersubstanzen, Salze, Detergenzien, Ionen, insbesondere Calciumionen und Chelatbildner.

In einer bevorzugten Ausführungsform des erfindungsgemäßen Verfahrens zur Bestimmung der Aktivität eines proteolytischen Gerinnungsfaktors wird die Probe zusätzlich mit einem Fibrinaggregationshemmer vermischt. Fibrinaggregationshemmer sind Substanzen, die die Aggregation von Thrombin-induzierten Fibrinmonomeren verhindern. Auf diese Weise wird die Entstehung eines Fibringerinnsels in einer fibrinogenhaltigen Probe verhindert, welches ansonsten die Messung, beispielsweise durch Limitation der Diffusion und Quenching, negativ beeinträchtigen könnte. Bevorzugte Fibrinaggregationshemmer sind synthetische Peptide, wie z.B. ein Peptid der Sequenz Glycin-Prolin-Arginin-Prolin (kommerziell erhältlich als Pefabloc®FG, Pentapharm, Schweiz). Weitere bevorzugte Peptide, die als Fibrinaggregationshemmer verwendet werden können, insbesondere das bevorzugte Peptid der Sequenz Glycin-Prolin-Arginin-Prolin-Alanin, sind in EP-A2-456152 beschrieben.

Nachdem das Reaktionsgemisch, das die Probe, ein Substrat, welches von dem zu bestimmenden Enzym gebunden und modifiziert werden kann, und eine erste und zweite Komponente eines signalbildenden Systems enthält, bereit gestellt ist, wird das Reaktionsgemisch für eine begrenzte Zeit inkubiert, um eine ausreichende Assoziation von Enzym und Substrat zu gewährleisten. Der Begriff "ausreichend" ist so zu verstehen, dass das Verfahren als Ganzes eine quantitative Bestimmung der Aktivität des Enzyms ermöglicht. Die optimale Inkubationsdauer eines bestimmten Testaufbaus kann experimentell ermittelt werden. Das Signal bzw. die Veränderung des Signals mit der Zeit, welches im Reaktionsgemisch entsteht, korreliert mit der Aktivität des nachzuweisenden Enzyms oder einem Aktivitätsmodulator. Über diese Korrelation kann mit Hilfe des Signals bzw. der zeitlichen Änderung des Signals die Aktivität eines Enzyms ermittelt werden. Hierfür wird vorzugsweise eine Kalibration mit einem Standard durchgeführt. Der Standard besitzt eine definierte Aktivität oder Konzentration des Enzyms oder des Aktivitätsmodulators.

Der vorliegenden Erfindung lag ferner die Aufgabe zugrunde, ein homogenes Verfahren bereit zu stellen, das neben der spezifischen Bestimmung der Enzymaktivität die simultane Bestimmung der Enzymmenge im selben Reaktionsgemisch ermöglicht.

Diese Aufgabe wird dadurch gelöst, dass der zu untersuchenden Probe ferner eine dritte Komponente des signalbildenen Systems zugegeben wird, welche mit dem zu bestimmenden Enzym während der Inkubation assoziiert wird. Die dritte Komponente des signalbildenen Systems ist so beschaffen, dass ein zweites detektierbares Signal entsteht, welches von dem ersten Signal unterschiedlich ist und welches nur entsteht, wenn die dritte und die zweite Komponente des signalbildenden Systems durch die gleichzeitige Bindung an das zu bestimmende Enzym in räumliche Nähe zueinander gebracht werden. Das zweite enstehende Signal verhält sich proportional zur Enzymmenge in der Probe.

Ein Vorteil dieses Verfahrens zur simultanen Bestimmung von Enzymaktivität und -menge in ein und demselben Reaktionsgemisch besteht darin, dass in einem einzigen Testdurchgang festgestellt werden kann, ob die Ursache für eine z.B. gegenüber der Norm verminderte Enzymaktivität eine verminderte Enzymmenge ist oder ob die Enzymmenge der Norm entspricht und daher eine andere Ursache für die verminderte Enzymaktivität angenommen werden muss, wie z.B. die Anwesenheit eines Aktivitätsmodulators, wie eines Inhibitors, oder eine Funktionsstörung des Enzyms. Die simultane Bestimmung verschiedener Parameter in einem einzigen Reaktionsgemisch hat zusätzlich den Vorteil, dass etwaige Ungenauigkeiten bei der Pipettierung der Probe oder Ungenauigkeiten, die durch einen Alterungsprozess der Probe hervorgerufen werden könnten, vermieden werden. Damit ist eine genaue Bestimmung der spezifischen Enzymaktivität möglich.

Die dritte Komponente des signalbildenden Systems ist so beschaffen, dass ein zweites detektierbares Signal entsteht, welches von dem ersten Signal unterschiedlich ist und welches nur entsteht, wenn die dritte und die zweite Komponente des signalbildenden Systems durch die gleichzeitige Bindung an das zu bestimmende Enzym in räumliche Nähe zueinander gebracht werden. Bei der dritten Komponente des signalbildenden Systems kann es sich entweder um einen alternativen Energiespender oder um einen alternativen Energieempfänger handeln. Auch die dritte Komponente des signalbildenden Systems kann mit einer partikulären Festphase assoziiert sein.

In einer Ausführungsform der simultanen Bestimmung der Enzymmenge weist das zu bestimmende Enzym zusätzlich einen ersten Bindungspartner C eines dritten Bindungspaares C/D auf, wobei der zweite Bindungspartner D des dritten Bindungspaares C/D mit der dritten Komponente des signalbildenden Systems assoziiert ist und wobei das Enzym durch Bindung der Bindungspartner C und D während der Inkubation an die dritte Komponente des signalbildenden Systems gebunden wird. Das dritte Bindungspaar C/D muss sich von dem zweiten Bindungspaar X/Y unterscheiden.

Die Bindungspartner C und D sind zwei unterschiedliche Moleküle, die einander spezifisch erkennen und binden. C ist ein Enzym-spezifisches Struktur- oder Sequenzepitop und D ist -je nach Beschaffenheit des nachzuweisenden Enzymsein Antikörper oder Antikörperfragment oder ein Polynukleotid mit Spezifität für das Enzym-spezifische Struktur- oder Sequenzepitop.

In einer Ausführungsform ist beispielsweise die erste Komponente des signalbildenden Systems ein erstes chemilumineszierendes Agens, die zweite Komponente des signalbildenden Systems ist ein Photosensitizer, und die dritte Komponente ist ein zweites chemilumineszierendes Agens, wobei das erste und zweite chemilumineszierende Agens voneinander unterschiedliche Emissionsspektren aufweisen. Die Messung der beiden Lichtsignale mit unterschiedlichen Emmisionsspektren ermöglicht einerseits die quantitative Bestimmung der Enzymaktivität und die quantitative Bestimmung der Enzymmenge im selben Reaktionsgemisch.

In einer anderen Ausführungsform ist beispielsweise die erste Komponente des signalbildenden Systems ein erster Photosensitizer, die zweite Komponente des signalbildenden Systems ist ein chemilumineszierendes Agens ist und die dritte Komponente ist ein zweiter Photosensitizer, wobei der erste und zweite Photosensitizer durch Licht unterschiedlicher Wellenlänge anregbar sind. Die Messung des Lichtsignals nach Anregung des ersten Photosensitizers mit Licht einer Wellenlänge X ermöglicht die quantitative Bestimmung der Enzymaktivität. Die Messung des Lichtsignals nach Anregung des zweiten Photosensitizers mit Licht einer Wellenlänge Y ermöglicht die quantitative Bestimmung der Enzymmenge im selben Reaktionsgemisch.

Der vorliegenden Erfindung lag ferner die Aufgabe zugrunde, ein homogenes Verfahren bereit zu stellen, das die Bestimmung eines Enzymaktivitätsmodulators ermöglicht. Unter einem Enzymaktivitätsmodulator ist eine Substanz zu verstehen, die als Inhibitor, Aktivator, Agonist, Antagonist oder Cofaktor die Aktivität eines Enzyms zu beeinflussen (modulieren) vermag.

Diese Aufgabe wird dadurch gelöst, dass der zu untersuchenden Probe eine definierte Menge eines Enzyms, dessen Aktivität direkt oder indirekt von dem zu bestimmenden Enzymaktivitätsmodulator beeinflussbar ist, in Form eines separaten Reagenzes zugegeben wird. Ferner wird dem Reaktionsgemisch ein Substrat zugegeben, welches von dem zugegebenen Enzym gebunden und modifiziert werden kann, sowie eine erste und eine zweite Komponente eines signalbildenden Systems, wobei die erste Komponente des signalbildenden Systems mit dem Substrat assoziiert ist oder wird und wobei die zweite Komponente des signalbildenden Systems mit dem zugegebenen Enzym assoziiert ist. Das signalbildende System ist so beschaffen, dass ein detektierbares Signal nur entsteht, wenn die erste und die zweite Komponente des signalbildenden Systems durch Bindung des zu bestimmenden Enzyms an das Substrat in räumliche Nähe zueinander gebracht werden. Das gemessene Signal korreliert mit der Enzym-modulierenden Aktivität des Enzymmodulators in der Probe.

Das erfindungsgemäße Verfahren eignet sich sowohl zur Bestimmung von Enzym-Inhibitoren als auch zur Bestimmung von Enzym-Aktivatoren. Zur Bestimmung eines Enzym-Inhibitors wird das Enzym in aktivierter Form dem Reaktionsgemisch zugegeben. Zur Bestimmung eines Enzym-Aktivators wird das Enzym in nicht-aktivierter Form dem Reaktionsgemisch zugegeben.

Insbesondere eignet sich das erfindungsgemäße Verfahren zur Bestimmung von Antikoagulanzien, d.h. von Substanzen, die die Aktivität bestimmter Blutgerinnungfaktoren inhibieren. Für die Bestimmung eines Antikoagulanzes in einer Probe wird eine bekannte, definierte Menge eines aktivierten Gerinnungsfaktors zu dem Reaktionsgemisch gegeben. Welcher aktivierte Gerinnungsfaktor zugegeben wird, hängt davon ab, welches Antikoagulanz bestimmt werden soll.

Für die Bestimmung eines Heparins, d.h. eines hochmolekularen, unfraktionierten Heparins (HMW Heparin) oder eines niedermolekularen Heparins (LMW Heparin) oder eines Heparinoids, eignet sich insbesondere die Zugabe von Faktor IIa (Thrombin) oder von Faktor Xa. Für die Bestimmung eines direkten Thrombininhibitors, z.B. von Argatroban, Melagatran, Ximelagatran, Bivalirudin, Dabigatran oder Hirudin, eignet sich insbesondere die Zugabe von Faktor IIa (Thrombin). Für die Bestimmung eines direkten Faktor Xa-Inhibitors, z.B. von Rivaroxaban, eignet sich insbesondere die Zugabe von Faktor Xa.

Abgesehen davon, dass bei dem erfindungsgemäßen Verfahren zur quantitativen Bestimmung eines Enzymmodulators eine definierte Menge eines Enzyms, dessen Aktivität direkt oder indirekt von dem zu bestimmenden Enzymmodulator beeinflussbar ist, und wobei das Enzym in einem separaten Reagenz enthalten ist, welches dem Reaktionsgemisch zugegeben wird, treffen die obigen Erläuterungen zur Durchführung des Verfahrens zur Bestimmung der Aktivität eines Enzyms auch auf das erfindungsgemäße Verfahren zur quantitativen Bestimmung eines Enzymmodulators zu.

Die Messung der enzymatischen Reaktion kann über das komplette Zeitintervall der Reaktion bis zur Gleichgewichtseinstellung oder in mindestens einem bestimmten Zeitintervall oder zu mindestens einem Zeitpunkt erfolgen. Zur Bestimmung der Aktivität können die Messwerte an sich oder in Bezug auf einen Zeitintervall verwendet werden. Wird die Aktivität durch photometrische Daten in Bezug auf einen Zeitintervall bestimmt, d.h. Umsatz- bzw. Reaktionsgeschwindigkeit erfasst, können zur Bestimmung der Umsatz- bzw. Reaktionsgeschwindigkeit verschiedene Verfahren eingesetzt werden. Beispielsweise kann die Umsatzgeschwindigkeit mittels Zeit-Umsatz-Kurven bestimmt werden. Bei Zeit-Umsatz-Kurven wird die Umsetzung des Substrates gegenüber der Zeit aufgetragen. Zur Bestimmung der Umsatzgeschwindigkeit wird eine Gerade im Bereich der Reaktion nullter Ordnung der Zeit-Umsatz-Kurve, üblicherweise zu Beginn einer Messung einer Enzymreaktion, angelegt. Die Steigung der Gerade liefert dann die Umsatzgeschwindigkeit, d.h. die Änderung der Konzentration des Substrates oder Produktes in einem bestimmten Zeitintervall.

Messgrößen bzw. Parameter, die sich für die Auswertung der Umsatzkinetik eignen, sind z.B. alle Parameter, die die Reaktionskinetik beschreiben, wie z.B. die Kurvendiskussion per se, insbesondere jedoch einzelne Parameter der Reaktionskinetik, wie die maximale Steigung, d.h. die Reaktionsgeschwindigkeit (vₘₐₓ), Sigmoiditätsparameter, Linearitätsparameter, die Fläche unter der Kurve, etc. Parameter, die sich für die Testauswertung eignen, sind z.B. auch absolute Messwerte, wie z.B. LOCI-Messwerte, die zu einem bestimmten Zeitpunkt gemessen werden oder ein Zeitpunkt, zu dem ein bestimmter Messwert, z.B. ein Maximalwert erreicht ist.

Bei dem Umsatz eines Substrates reagieren Enzyme mit ihren Substraten unter geeigneten Bedingungen und bilden einen Enzym-Substrat-Komplex. Sind die Bedingungen entsprechend gewählt, so kann es in einem weiteren Schritt zur Umwandlung des Substrates in mindestens ein Produkt kommen.

Aufgrund der Verwendung eines "signalbildendes Systems" sind verschiedene zeitliche Abhängigkeiten des Signals bei der Bestimmung von Enzymaktivitäten nutzbar.

Wenn der Bindungspartner des signalbildenden Systems für das Enzym in ausreichender Konzentration eingesetzt wird, kann die Konzentration und damit die Aktivität von freiem Enzym (FE) vernachlässigt werden, wenn sich das System im Gleichgewicht befindet. Ansonsten kann sich zumindest zu Beginn der Reaktion die Bildung des Komplexes aus Enzym und Bindungspartner des Enzyms auf das Signal auswirken. Am Bindungspartner gebundenes Enzym (GE) kann mit Substrat reagieren. Bei dem Substrat sind freies Substrat (FS) und an den entsprechenden Bindungspartner des signalbildenden Systems gebundenes Substrat (GS) zu unterscheiden. GE bindet demnach entweder FS oder GS, d.h. S und FS konkurrieren miteinander um die Bindung am GE. Entsprechendes gilt für den Umsatz von FS oder GS. Hierbei ist zu berücksichtigen, dass FS und GS nicht unbedingt die gleiche Affinität zu GE haben müssen. Die Bindung eines Substrates an das signalbildende System kann zu einer Affinitätsänderung führen. Wenn die Affinität von FS höher als die von GS ist, wird mehr FS von GE gebunden und umgekehrt. Dies trifft auch auf die Umsetzung des FS und GS in ein Produkt zu. Die Reaktivität bzw. Umsetzbarkeit in ein Produkt eines Substrates kann sich mit der Bindung an das signalbildende System ändern, d.h. erhöhen oder erniedrigen. Es ist denkbar, dass sich die Umsetzungsreaktion eines Substrates nach der Bindung des Substrates an das signalbildende System derart erniedrigt, dass die Umsetzungsreaktion des gebundenen Substrates gegenüber der Bindungsreaktion vernachlässigt werden kann oder dass das Substrat in der gebundenen Form kaum oder nicht mehr umgesetzt wird.

Da bei der Verwendung von LOCI als signalbildendem System nur die Bindung von GS an GE ein Signal liefert, beeinflusst die Kompetition von FS und GS das Signal oder dessen Kinetik. Die Kompetition hängt von der jeweiligen Konzentration, Affinität oder Umsetzbarkeit von FS und GS ab.

Wird die Menge an Substrat so gewählt, dass überwiegend GS vorliegt, kann die Konzentration an FS vernachlässigt werden. FS spielt dann bei der Signalbildung kaum eine Rolle. Das heißt, die Konzentration, Affinität und Umsetzbarkeit von GS beeinflusst dann das Signal oder dessen Kinetik maßgeblich. Überwiegt die Umsetzungsreaktion von GS die Bindungsreaktion, so ist mit einer Abnahme des Signals mit der Zeit zu rechnen. Überwiegt hingegen die Bindungsreaktion die Umsetzungsreaktion, so ist zunächst mit einer Signalzunahme mit der Zeit zu rechnen. Das Ausführungsbeispiel 1 gibt diese Situation wieder. Bei ausreichender Umsetzungsreaktion kann es ggf. zu einem späteren Zeitpunkt wieder zu einer Signalabnahme mit der Zeit kommen, wenn die Bindung sich dem
Gleichgewichtszustand nähert.

Die folgenden Beispiele dienen der Veranschaulichung der vorliegenden Erfindung und sind nicht als Einschränkung zu verstehen.

### BEISPIELE

### Materialien

Sofern nicht anders angegeben, sind alle Reagenzien von Sigma Aldrich Corp. (St. Louis, MO, USA).

### Substrate

Folgende FSAP-sensitiven Peptide wurden nach dem Fmoc Peptidsynthesestandardverfahren hergestellt:
1. ein Pentapeptid, gekoppelt an Biotin mit den Aminosäureresten Arginin, Prolin, Arginin, Phenylalanin, Lysin in der genannten Sequenz und einem Biotin am C-terminalen Lysinrest (Peptid I);
2. ein Tetrapeptid, gekoppelt an Biotin mit den Aminosäureresten Isoleucin, Prolin, Arginin, Lysin in der genannten Sequenz und einem Biotin am C-terminalen Lysinrest (Peptid II);
3. ein Peptid nach 1 oder 2 mit einem Polyethylenglykol mit 12 Ethylenglykoleinheiten (PEG₁₂ Spacer) zwischen dem N-terminalen Lysinrest und dem Biotin (Peptide III nach 1 und Peptid IV nach 2).

### Signalbildendes System

Das hier verwendete signalbildende System basiert auf der bekannten LOCI Technologie (Luminescent oxygen channeling assay, siehe auch EP-A2-0515194). Das System umfasst einen Photosensitizer und ein chemilumineszierendes Agens. Die Anregung des Photosensitizers mit Licht verursacht die Entstehung von kurzlebigem Singulettsauerstoff, welcher das chemilumineszierende Agens zu aktivieren vermag, wodurch ein Lumineszenzsignal emittiert wird.

### Chemibeads

Die erste Komponente des signalbildenden Systems wird im Folgenden als Chemibead bezeichnet. Es umfasst das chemilumineszierende Agens, welches mit einer partikulären Latexfestphase assoziiert ist. Chemibeads wurden mit einem monoklonalen anti-FSAP-Antikörper (Siemens Healthcare Diagnostics Products GmbH, MAk Nr.1102-677(26)), der von der Hybridomazelllinie DSM ACC2453 gebildet wird (siehe EP-A1-1650305), nach folgendem Verfahren konjugiert: Der Antikörper wurde über eine Sephadex® G-25-Säule in 300 mM NaCl, 0,05 % Tween® 20, 10 mM Phosphatpuffer, pH 7,0 umgepuffert und auf 20 mg/mL ankonzentriert. 4 mg Antikörper wurden an 20 mg Chemibeads unter Zusatz von 25 mg/mL NaCNBH3 kovalent gekoppelt. Nach einem Inkubationsschritt und Blockierung noch freier Reaktionspartner wurden die Antikörper-konjugierten Chemibeads über Diafiltration gereinigt und anschließend in 50 mM HEPES, 300 mM NaCl, 1 mM EDTA, 0,1 % Triton® X-405, 1 mg/mL BSA, 0,15 % PROCLIN® 300, 0,1 mg/mL Neomycinsulfat, pH 8,0 aufgenommen.

### Sensibeads

Die zweite Komponente des signalbildenden Systems wird im Folgenden als Sensibead bezeichnet. Es umfasst den Photosensitizer (Trihexyl)-Silicon-t-Butyl-Phtalocyanin, welcher mit einer partikulären Latexfestphase assoziiert ist, die ferner Streptavidin-beschichtet ist.

### Faktor VII-aktivierende Protease (FSAP)

Two-chain FSAP (tc-FSAP) wurde hergestellt, wie von Kannemeier et al. beschrieben (Kannemeier, C. et al. (2001) Factor VII and single-chain plasminogen activatoractivating protease: Activation and autoactivation of the proenzyme. Eur. J. Biochem. 268 (13): 3789-3796).

### Beispiel 1: Erfindungsgemäßes Verfahren zur Bestimmung der FSAP-Aktivität

Die weiter oben beschriebenen FSAP-sensitiven Peptide I-IV, wurden in Aqua Dest. in einer Konzentration von 2 mg/mL (Konzentrat) gelöst und für den Test auf ca. 20 µg/mL in Testpuffer (25 mM HEPES, 140 mM NaCl, 10 mM CaCl₂, 1 % Tween® 20, 1 % BSA und 1 % Dextran T-500, pH 6,3) verdünnt. Die Chemibeads wurden auf 200 µg/mL und die Sensibeads auf 400 µg/mL in Testpuffer verdünnt.

### Testansatz I:

120 µL Testpuffer wurden gemischt mit 10 µL Anti-FSAP Chemibeads, 10 µL gereinigter tc-FSAP in Testpuffer (10 µg/mL), 10 µL biotinyliertem Peptidsubstrat und 10 µL Sensibeads und in DIMENSION VISTA® Reaktionsküvetten (Siemens Healthcare Diagnostics Products GmbH, Marburg, Deutschland) 10 Minuten bei 37 °C inkubiert. Im Anschluss erfolgte eine kinetische Messung der Chemilumineszenz des Reaktionsansatzes, im Folgenden auch LOCI® Signal genannt, über 30 Minuten in einer kommerziell erhältlichen DIMENSION VISTA® LOCI® Messeinheit (Siemens Healthcare Diagnostics Products GmbH). Pro Messpunkt wurde der Reaktionsansatz für 200 ms mit Licht einer Wellenlänge von 680 nm illuminiert und das entstehende LOCI® Signal über 1000 ms bei 612 nm gemessen.

In einer Versuchsreihe wurde die Signalbildung bei Verwendung der vier weiter oben beschriebenen Peptidsubstrate I-IV untersucht. Tabelle 1 zeigt die Signalbildung in Kilocounts (kcounts) der vier untersuchten Peptidsubstrate in Gegenwart des aktiven Enzyms und ohne Enzym. Nur in Gegenwart des aktiven Enzyms zeigten alle vier untersuchten biotinylierten Peptide eine deutliche Signaldynamik über 30 Minuten. Ist kein Enzym vorhanden, ist auch keine signifikante Änderung des Signals mit der Zeit erkennbar.

**Tabelle 1**

| **Testansatz** | Messkinetik [Min.] | Peptid I [kcounts] | Peptid II [kcounts] | Peptid III [kcounts] | Peptid IV [kcounts] |
|---|---|---|---|---|---|
| Testansatz I (Alle Komponenten) | 2 | 25,013 | 23,352 | 18,237 | 17,364 |
| | 5 | 37, 83 | 35,704 | 29,534 | 26,484 |
| | 10 | 59,635 | 54, 607 | 49,056 | 45,347 |
| | 15 | 85,131 | 79,409 | 72,159 | 66,918 |
| | 30 | 144,879 | 128,201 | 88,234 | 81,497 |
| | | | | | |

| **Negative Kontrolle** | Messkinetik [Min.] | Peptid I [kcounts ] | Peptid II [kcounts] | Peptid III [kcounts] | Peptid IV [kcounts] |
|---|---|---|---|---|---|
| Testansatz I (ohne tc-FSAP) | 2 | 15,456 | 13, 875 | 1,946 | 2, 877 |
| | 5 | 16,814 | 14, 672 | 2,231 | 3,214 |
| | 10 | 16,478 | 16,427 | 2,433 | 3,544 |
| | 15 | 17,127 | 16,127 | 2,412 | 3,724 |
| | 30 | 16,972 | 16,423 | 2,573 | 3,709 |

Neben dem einfachen Nachweis eines aktiven Enzyms kann dieses auch quantitativ bestimmt werden. In Tabelle 2 ist die Abhängigkeit des LOCI-Signals von der Konzentration aktiver FSAP (Zweikettenform; two-chain (tc)) dargestellt. Zur Bestimmung der FSAP-Aktivität wurde eine tc-FSAP Verdünnungsreihe in Testpuffer (18 bis 600 plasmaäquivalente Units (PEU)/mL bzw. 1,5 bis 50 µg/mL) bei einer Peptidsubstratkonzentration von 20 µg/mL, wie im Testansatz I beschrieben, getestet. Gemessen wurde nach 30minütiger Inkubation bei 37 °C. Tabelle 2 zeigt, dass die Konzentration und die damit einhergehende Aktivität der FSAP mit dem Signal korreliert und damit auch quantifiziert werden kann. Die Quantifizierung kann beispielsweise über eine Kalibration mit einem entsprechenden Standard aktiver FSAP mit bekannter Aktivität erfolgen. Wird im Ansatz keine tc-FSAP oder kein Peptidsubstrat zugegeben, so kommt es nicht zum Signalaufbau (Tabelle 3).

**Tabelle 2**

| **tc-FSAP** | **tc-FSAP** | **Signal** |
|---|---|---|
| **[µg/mL]** | **[PEU/mL]** | **[kcounts]** |
| 0 | 0 | 2,735 |
| 1,5 | 18 | 51,4 |
| 3 | 36 | 80,146 |
| 6 | 72 | 101,731 |
| 12,5 | 150 | 116, 522 |
| 25 | 300 | 146,279 |
| 50 | 600 | 215,144 |

**Tabelle 3**

| **tc-FSAP** | **Peptidsubstrat** | **Signal** |
|---|---|---|
| **[µg/mL]** | **[µg/mL]** | **[kcounts]** |
| 12,5 | 0 | 2,391 |
| 0 | 20 | 2,735 |
| 12,5 | 20 | 116, 522 |

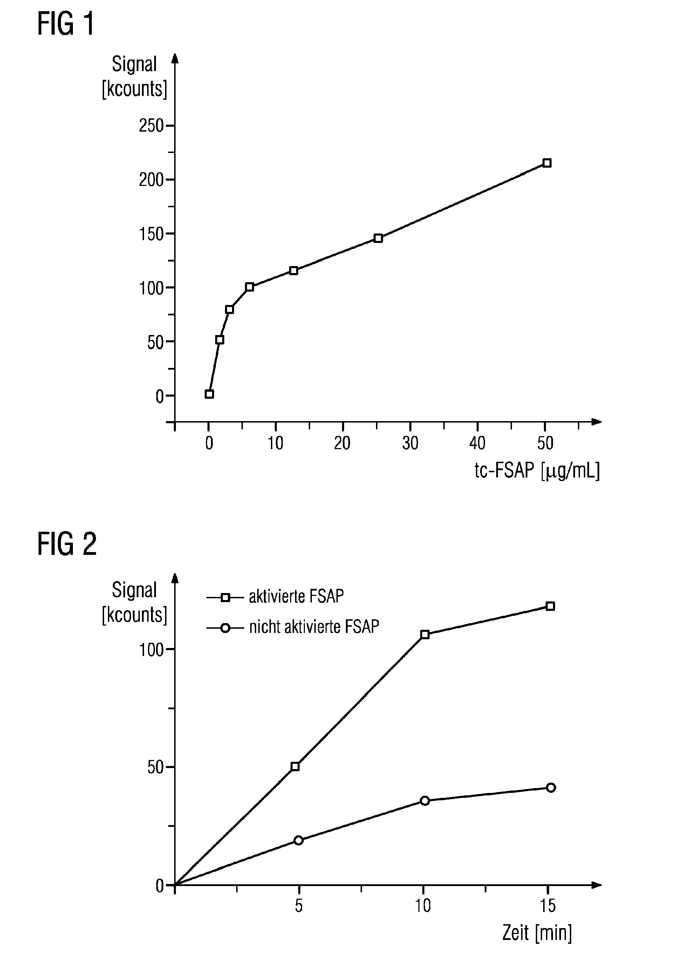

In Abbildung 1 ist die Abhängigkeit des LOCI-Signals von der Enzymaktivität bzw. -konzentration basierend auf den Daten von Tabelle 2 dargestellt. Interessant ist die biphasische Abhängigkeit. Bei geringen FSAP-Konzentrationen ist ein starker Signalanstieg zu verzeichnen. Bei höheren Konzentrationen ist dieser weniger stark. Es kann davon ausgegangen werden, dass im niedrigen Konzentrationsbereich der Anstieg der Enzymkonzentration und damit der Anteil des gebundenen Enzyms in höherem Maße zur Signalbildung beiträgt als bei höheren Konzentrationen. Im höheren Konzentrationsbereich des Enzyms hingegen trägt die Bindungsreaktion oder Umsetzungsreaktion in höherem Maße zum Signal bzw. zur Signaländerung bei.

Die Antigen-Konzentration eines Enzyms muss nicht notwendigerweise mit der Aktivität des Enzyms korrelieren. Da die FSAP beispielsweise im Plasma sowohl als inaktives Zymogen (Einkettenform, single-chain (sc)) wie auch aktives Enzym (tc-FSAP) zirkuliert, ist eine hundertprozentige Korrelation zwischen der FSAP-Antigenkonzentration und der FSAP nicht notwendigerweise gewährleistet. Mit dem erfindungsgemäßen Verfahren kann sowohl die Aktivität als auch die Menge des Enzyms ermittelt werden. Damit kann auch die spezifische Aktivität eines Enzyms erfasst werden.

Um dies zu verdeutlichen, wurde Citratplasma zur Aktivierung der sc-FSAP 15 Minuten in Testpuffer mit
20 µg/mL hochmolekularem Dextransulfat
(Dextransulfatnatriumsalz von Leuconostoc spp., mittlere Molmasse ≥ 500 kDa) vorinkubiert und im Vergleich zu einer Probe, bei der keine Zugabe von hochmolekularem Dextransulfat zur Aktivierung der FSAP erfolgte, getestet. Eine Aktivierung in Anwesenheit von Dextransulfat führt zu einer anderen Signalhöhe und Signaldynamik im Gegensatz zu derselben Probe, mit derselben FSAP-Konzentration, bei der keine Aktivierung durch Dextransulfat erfolgte (Tabelle 4 und Abbildung 2). Die durch Aktivierung der FSAP erzeugte Signalhöhe entspricht etwa dem Signal einer gleichkonzentrierten, gereinigten tc-FSAP von 12 µg/mL mit einer Enzymaktivität von 1 PEU/mL (nicht dargestellt).

**Tabelle 4: Einfluss von Dextransulfat (DXS) als Cofaktor der Aktivierung von FSAP in Plasma**

| **Inkubations-zeit** | **Testpuffer mit DXS (20 µg/mL, MW ≥500 kDa)** | | **Testpuffer ohne DXS** | |
|---|---|---|---|---|
| | Peptidkonzentration [µg/mL] | | Peptidkonzentration [µg/mL] | |
| | **10** | **0** | **10** | **0** |
| bei +37°C | Signal [kcounts] | Signal [kcounts] | Signal [kcounts] | Signal [kcounts] |
| **5** | 50,303 | 1, 166 | 19,359 | 0,856 |
| **10** | 106,500 | 1,220 | 36,009 | 1, 005 |
| **15** | 118,096 | 1,204 | 41, 630 | 0,823 |

Wie zuvor ausgeführt, kann davon ausgegangen werden, dass der Einfluss auf die Signalbildung der Enzymaktivität im Vergleich zur Enzymkonzentration mit fortlaufender Reaktionszeit zunimmt. Dies wird in Abbildung 2 deutlich.

Das Verhältnis der Signale bei 15 Minuten zu 5 Minuten Inkubation beträgt bei der aktivierten FSAP ca. 2,35, bei der nicht aktivierten FSAP ca. 2,15. Wenn davon ausgegangen wird, dass die Enzymaktivität zu einem späteren Zeitpunkt einen größeren Beitrag zur Signalbildung leistet als die Enzym- bzw. Antigenkonzentration, ist das höhere Signalverhältnis von 2,35 gegenüber 2,15 ein Indikator für eine höhere spezifische Aktivität der FSAP in der aktivierten Probe gegenüber der nicht aktivierten Probe.

Analog verhält es sich mit dem Verhältnis der Signale von aktivierter FSAP zu nicht aktivierter FSAP bei 15 und 5 Minuten von 2,84 und 2,6. Das höhere Signalverhältnis ist wiederum eine Messgröße für die höhere spezifische Aktivität der FSAP in der aktivierten Probe gegenüber der nicht aktivierten Probe.

Ebenso können andere Parameter der Abhängigkeit des Signals von der Zeit als Messgröße für die Antigen- bzw. Enzymkonzentration und die Enzymaktivität und die daraus resultierende spezifische Aktivität verwendet werden. Beispielsweise liefert die Steigung bei der aktivierten FSAP bis 10 Minuten einen Wert von 10,65 kcounts/min und nach 10 Minuten 2,32 kcounts/min.

Die Steigung bei der nicht aktivierten FSAP bis 10 Minuten liefert einen Wert von 3,60 kcounts/min und nach 10 Minuten 1,12 kcounts/min.

Die Reduktion der Signalzunahme mit der Zeit (Steigung) nach 10 Minuten ist bei der aktivierten FSAP deutlich höher als bei der nicht aktivierten FSAP. Die Reduktion kann beispielsweise durch das Verhältnis oder die Differenz der Steigungen quantifiziert werden, d.h. ca. 3 gegenüber 2 und 7 gegenüber 1,2. Der höhere Umsatz des gebundenen Substrates durch die aktivere FSAP, im Vergleich zur inaktiveren FSAP führt vermutlich zu einer deutlich reduzierten Zunahme des Signals mit der Zeit. Prinzipiell können auch andere Parameter einer Kurvendiskussion zur Ermittlung der Antigenkonzentration, der Enzymaktivität oder der spezifischen Aktivität herangezogen werden.

### Beispiel 2: Erfindungsgemäßes Verfahren zur Bestimmung eines Inhibitors einer Enzymaktivität

Mit der Bestimmung der spezifischen Aktivität eines Enzyms kann man auch Informationen über das mögliche Vorhandensein von Aktivitätsmodulatoren bekommen. Das erfindungsgemäße Verfahren erlaubt aber auch die Quantifizierung des Einflusses und damit auch die quantitative Bestimmung von Aktivitätsmodulatoren. Dies ist im Folgenden am Beispiel eines Inhibitors der FSAP, Aprotinin, gezeigt. In Abhängigkeit der Konzentration des Inhibitors ändert sich das Signal bzw. dessen Verlauf. Die Kinetik der Enzym-Inhibitor-Wechselwirkung wurde durch Messungen der Enzymaktivität in Abhängigkeit von der Hemmstoffmenge untersucht. In den tc-FSAP-Inhibitionsansätzen wurden konstante Enzymmengen (tc-FSAP, 10 µg/mL) mit variablen Inhibitorkonzentrationen (Aprotinin aus Rinderlunge, Fluka) vorinkubiert, um die Ausbildung des Enzym-Inhibitor-Komplexes zu ermöglichen. Ein weiterer Ansatz erfolgte ohne Vorinkubation. Die verbleibende Proteaseaktivität wurde nach der Peptidsubstratzugabe, wie unter Beispiel 1 beschrieben, bestimmt.

Testansatz:
120 µL Testpuffer pH 6,3, 10 µL Anti-FSAP Chemibeads (200 µg/mL in Testpuffer), 10 µL gereinigte tc-FSAP (10 µg/mL) und 10 µL Testpuffer mit 0, 4,7 und 47,2 U/mL Aprotinin wurden in DIMENSION VISTA® LOCI® Reaktionsküvetten gemischt. Ohne Vorinkubation oder nach 10 Minuten Vorinkubation bei 37 °C erfolgte die Zugabe von 10 µL biotinyliertem Peptidsubstrat IV in Testpuffer und 10 µL Sensibeads (400 µg/mL in Testpuffer). Das entstehende LOCI® Signal wurde in einer kommerziell erhältlichen DIMENSION VISTA® LOCI® Messeinheit (Siemens Healthcare Diagnostics Products GmbH) mit 200 ms Illumination bei 680 nm und 1000 ms Messzeit bei 612 nm, kinetisch, über 15 Minuten bei 37 °C gemessen. Aprotinin zeigt einen stark signalreduzierenden Effekt, vor allem nach einer Vorinkubation mit tc-FSAP (Tabelle 5).

**Tabelle 5: Inhibition der tc-FSAP Aktivität mit Aprotinin ohne und mit Vorinkubation; Messwert nach 15 Minuten**

| **Aprotinin [U/mL]** | **Ohne Vorinkubation Signal [kcounts]** | **Mit 10 Min. Vorinkubation Signal [kcounts]** |
|---|---|---|
| 0 | 117,27 | 117,27 |
| 4,7 | 71,18 | 15,03 |
| 47,3 | 67, 8 | 11,77 |

Als weiterer Hemmstoff wurde ein die FSAP-Aktivität inhibierender monoklonaler Antikörper (Siemens Healthcare Diagnostics Products GmbH, MAk Nr.1102/570), der gegen die leichte Kette der FSAP gerichtet ist und von der Hybridomazelllinie DSM ACC2533 gebildet wird (siehe EP-A2-1334983), analog dem Ansatz mit Aprotinin eingesetzt. Tabelle 7 zeigt den steigenden inhibitorischen Effekt mit zunehmender Konzentration des eingesetzten monoklonalen Antikörpers.

**Tabelle 7: Inhibition der tc-FSAP Aktivität mit Anti-FSAP MAk 1102/570**

| **Anti-FSAP Mak 1102/570 [µg/mL]** | **Ohne Vorinkubation Signal [kcounts]** |
|---|---|
| 0 | 86,5 |
| 24 | 10,3 |
| 240 | 2,7 |

Sowohl beim Aprotinin als auch beim inhibitorischen Antikörper verändert sich das Signal mit der jeweiligen Konzentration des Aktivitätsmodulators. Bei entsprechender Kalibrierung erlaubt dies die Quantifizierung des Aktivitätsmodulators. Der Inhibitor beeinflusst die Bindung oder die Umsetzung eines Substrates; des gebundenen Substrates oder des nicht gebundenen Substrates. Dominiert im gewählten Messsystem die Bindung oder der Bindungsprozess des gebundenen Enzyms an das gebundene Substrat, kommt es mit zunehmender Inhibitorkonzentration zu einer Abnahme des absoluten Signals oder der Reduktion der Zunahme mit der Zeit wie im Ausführungsbeispiel dargestellt. Dominiert hingegen die Umsetzungsreaktion kann es zu einer Erhöhung des Signals bzw. einer Abnahme der Reduktion des Signals mit der Zeit mit zunehmender Inhibitorkonzentration kommen.

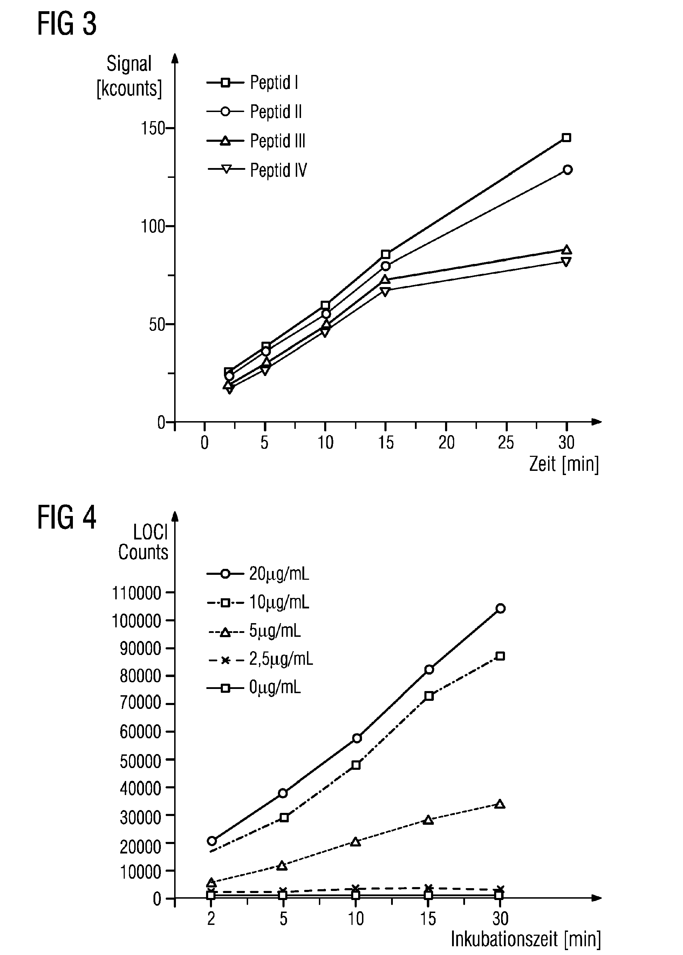

Das erfindungsgemäße Verfahren erlaubt außerdem die Bestimmung der Eigenschaften von Substraten. Dies wird Anhand von Abbildung 3 deutlich. Die Signale oder die Signaldynamiken sind auch von der Art der Substrate abhängig.

Dies ist möglicherweise auf eine unterschiedliche Bindungsreaktion und/oder Umsetzungsreaktion zurückzuführen. Der Signalanstieg bis 15 Minuten ist bei den vier verschiedenen Peptiden vergleichbar. Dies kann darauf hindeuten, dass die Bindungskinetik vergleichbar ist. Nach einer Inkubation von 30 Minuten sind die Signale im Vergleich zu Peptid I von Peptid II und insbesondere den Peptiden III und IV jedoch deutlich niedriger. Da vergleichbare Peptidkonzentrationen eingesetzt wurden, deutet dies ggf. auf unterschiedliche Affinitäten oder eine unterschiedliche Umsetzung der Peptide hin. Beispielsweise kann die Umsetzung des an die Sensibeads gebundenen Peptids I durch die Bindung an Sensibeads stark eingeschränkt sein. Damit wird das Signal insbesondere von der Bildung des Komplexes aus gebundenem Enzym und gebundenem Substrat bestimmt. Die Umsetzung des gebundenen Substrates ist in diesem Fall reduziert oder nicht möglich. Wird das gebundene Substrat jedoch umgesetzt, kann das Signal dann nicht mehr so stark zunehmen, da nun der Anteil an gebundenem Produkt zunimmt, das weniger oder nicht mehr vom Enzym gebunden wird. Damit wird das LOCI Signal reduziert. Somit kann davon ausgegangen werden, dass insbesondere die gebundenen Peptide III und IV besser umgesetzt werden als die Peptide I und II. Es ist denkbar, dass Substrate, nachdem diese an das Sensibead gebunden haben, nicht mehr vom Enzym gebunden oder gar umgesetzt werden können. An das Sensibead gebundene Substrate, die nicht mehr vom Enzym gebunden werden sind ungeeignet. Gebundene Substrate, die zwar kaum noch oder nicht mehr messbar umgesetzt werden, aber Enzym binden, sind noch geeignet.

Neben der Bestimmung der Aktivität und Konzentration eines Enzyms und unterschiedliche Verhalten mit verschiedenen Substraten, soll an einem weiteren Beispiel verdeutlicht werden, dass auch die Konzentration von Substraten bestimmt werden kann. In Abbildung 4 ist die Kinetik des LOCI Signals in Abhängigkiet von der verwendeten Peptidkonzentration dargestellt. Mit zunehmender Peptidkonzentration nimmt die Steigung der Signalkinetik zu.

## Patentansprüche

1. Verfahren zur Bestimmung der Aktivität eines Enzyms in einer Probe, welches folgende Schritte umfasst:
a) Bereitstellen und Inkubation eines Reaktionsgemisches enthaltend
i) die Probe,
ii) mindestens ein Substrat, welches von dem zu bestimmenden Enzym gebunden und umgesetzt werden kann,
iii) eine erste und eine zweite Komponente eines signalbildenden Systems, welche so zusammenwirken, dass ein detektierbares Signal entsteht, wenn die erste und die zweite Komponente des signalbildenden Systems in räumliche Nähe zueinander gebracht werden und wobei das Substrat mit der ersten Komponente des signalbildenden Systems assoziiert ist oder während der Inkubation assoziiert wird und wobei das zu bestimmende Enzym mit der zweiten Komponente des signalbildenden Systems während der Inkubation assoziiert wird; und
b) Messung des Signals, welches mit der Enzymaktivität in der Probe korreliert.

2. Verfahren nach Anspruch 1, wobei das Substrat einen ersten Bindungspartner A eines ersten Bindungspaares A/B aufweist und wobei die erste Komponente des signalbildenden Systems den zweiten Bindungspartner B des ersten Bindungspaares A/B aufweist und wobei das Substrat durch Bindung der Bindungspartner A und B an die erste Komponente des signalbildenden Systems gebunden ist oder während der Inkubation gebunden wird.

3. Verfahren nach Anspruch 2, wobei die Bindungspartner A und B so gewählt sind, dass sie ein Bindungspaar A/B aus der Gruppe FLAG-Tag/anti-FLAG-Tag-Antikörper, HIS-Tag/anti-HIS-Tag-Antikörper, Fluorescein/anti-Fluorescein-Antikörper, Biotin/Avidin und Biotin/Streptavidin bilden.

4. Verfahren nach einem der vorherigen Ansprüche, wobei das zu bestimmende Enzym einen ersten Bindungspartner X eines zweiten Bindungspaares X/Y aufweist und wobei der zweite Bindungspartner Y des zweiten Bindungspaares X/Y mit der zweiten Komponente des signalbildenden Systems assoziiert ist und wobei das Enzym durch Bindung der Bindungspartner X und Y während der Inkubation an die zweite Komponente des signalbildenden Systems gebunden wird.

5. Verfahren nach Anspruch 4, wobei die Bindungspartner X und Y so gewählt sind, dass X ein Enzym-spezifisches Struktur- oder Sequenzepitop ist und Y ein Antikörper oder Antikörperfragment mit Spezifität für das Enzym-spezifische Struktur- oder Sequenzepitop.

6. Verfahren nach einem der vorherigen Ansprüche, wobei die erste Komponente des signalbildenden Systems mit einer ersten partikulären Festphase assoziiert ist und die zweite Komponente des signalbildenden Systems mit einer zweiten partikulären Festphase assoziiert ist.

7. Verfahren nach einem der vorherigen Ansprüche, wobei die erste Komponente des signalbildenden Systems ein chemilumineszierendes Agens ist und die zweite Komponente des signalbildenden Systems ein Photosensitizer ist oder umgekehrt.

8. Verfahren nach einem der vorherigen Ansprüche zur Bestimmung der Aktivität eines Enzyms aus der Gruppe Hydrolasen, insbesondere Peptidasen wie zum Beispiel Serinproteasen, Glycosidasen, Glycosylasen und Nukleasen.

9. Verfahren nach einem der vorherigen Ansprüche zur Bestimmung der Aktivität eines Enzyms aus der Gruppe der Proteasen, insbesondere Serinproteasen.

10. Verfahren nach einem der Ansprüche 1-7 zur Bestimmung der Aktivität eines proteolytischen Gerinnungsfaktors aus der Gruppe Faktor II, Faktor VII, Faktor IX, Faktor X, Faktor XI, Faktor XII und Protein C, wobei dem Reaktionsgemisch zusätzlich ein Agens zur direkten oder indirekten Aktivierung des proteolytischen Gerinnungsfaktors in der Probe zugegeben wird.

11. Verfahren nach Anspruch 10, wobei ein Agens aus der Gruppe Thromboplastin, Faktor IIa, Faktor VIIa, Faktor IXa, Faktor Xa, Faktor XIa, Faktor XIIa, aktiviertes Protein C, Schlangengifte, negativ geladene Phospholipide, Calciumionen, Gewebefaktor, Silica, Kaolin, Ellagsäure, Celite zur direkten oder indirekten Aktivierung des proteolytischen Gerinnungsfaktors verwendet wird.

12. Verfahren nach einem der Ansprüche 1-7 zur Bestimmung der Aktivität der Faktor VII-aktivierenden Protease.

13. Verfahren nach einem der vorherigen Ansprüche, wobei simultan, im selben Reaktionsgemisch die Menge des Enzyms bestimmt wird, zusätzlich umfassend folgende Schritte:
a) Zugabe einer dritten Komponente des signalbildenen Systems zum Reaktionsgemisch,
i) welche mit dem zu bestimmenden Enzym während der Inkubation assoziiert wird und
ii) welche so mit der zweiten Komponente des signalbildenden Systems zusammenwirkt, dass ein zweites detektierbares Signal entsteht, wenn die dritte und die zweite Komponente des signalbildenden Systems in räumliche Nähe zueinander gebracht werden, wobei das zweite Signal von dem Signal, das durch das Zusammenwirken der ersten und zweiten Komponente des signalbildenden Systems entsteht, unterschiedlich ist, und
b) Messung des zweiten Signals, welches mit der Enzymmenge in der Probe korreliert.

14. Verfahren nach Anspruch 13, wobei das zu bestimmende Enzym einen ersten Bindungspartner C eines dritten Bindungspaares C/D aufweist und wobei der zweite Bindungspartner D des dritten Bindungspaares C/D mit der dritten Komponente des signalbildenden Systems assoziiert ist und wobei das Enzym durch Bindung der Bindungspartner C und D während der Inkubation an die dritte Komponente des signalbildenden Systems gebunden wird.

15. Verfahren nach einem der Ansprüche 13 bis 14, wobei die erste Komponente des signalbildenden Systems mit einer ersten partikulären Festphase assoziiert ist und die zweite Komponente des signalbildenden Systems mit einer zweiten partikulären Festphase assoziiert ist und die dritte Komponente des signalbildenden Systems mit einer dritten partikulären Festphase assoziiert ist.

16. Verfahren nach einem der Ansprüche 13 bis 15, wobei die erste Komponente des signalbildenden Systems ein erstes chemilumineszierendes Agens ist und die zweite Komponente des signalbildenden Systems ein Photosensitizer ist und die dritte Komponente ein zweites chemilumineszierendes Agens ist, und wobei das erste und zweite chemilumineszierende Agens voneinander unterschiedliche Emissionsspektren aufweisen.

17. Verfahren nach einem der Ansprüche 13 bis 15, wobei die erste Komponente des signalbildenden Systems ein erster Photosensitizer ist und die zweite Komponente des signalbildenden Systems ein chemilumineszierendes Agens ist und die dritte Komponente ein zweiter Photosensitizer ist und wobei der erste und zweite Photosensitizer durch Licht unterschiedlicher Wellenlänge anregbar sind.

18. Verfahren zur quantitativen Bestimmung eines Enzymmodulators in einer Probe, wobei das Verfahren folgende Schritte umfasst:
a) Bereitstellen und Inkubation eines Reaktionsgemisches enthaltend
(i) die Probe,
(ii) eine definierte Menge eines Enzyms, dessen Aktivität direkt oder indirekt von dem zu bestimmenden Enzymaktivitätsmodulator beeinflussbar ist, und wobei das Enzym in einem separaten Reagenz enthalten ist, welches dem Reaktionsgemisch zugegeben wird,
(iii) ein Substrat, welches von dem zugegebenen Enzym gebunden und modifiziert werden kann,
(iv) sowie eine erste und eine zweite Komponente eines signalbildenden Systems, wobei die erste Komponente des signalbildenden Systems mit dem Substrat assoziiert ist oder wird und wobei die zweite Komponente des signalbildenden Systems mit dem zugegebenen Enzym assoziiert ist, wobei das signalbildende System so beschaffen ist, dass ein detektierbares Signal nur entsteht, wenn die erste und die zweite Komponente des signalbildenden Systems durch Bindung des zu bestimmenden Enzyms an das Substrat in räumliche Nähe zueinander gebracht werden, und
b) Messung des Signals, welches mit der Aktivität des Enzymmodulators in der Probe korreliert.

19. Verfahren nach Anspruch 18 zur Bestimmung eines Inhibitors eines Blutgerinnungsfaktors, wobei eine definierte Menge eines aktivierten Blutgerinnungsfaktors zugegeben wird.

20. Verfahren nach Anspruch 19 zur Bestimmung eines Inhibitors eines Blutgerinnungsfaktors aus der Gruppe Heparin, Argatroban, Melagatran, Ximelagatran, Bivalirudin, Dabigatran, Rivaroxaban und Hirudin.
